# EUROPEAN PATENT SPECIFICATION

(11) **EP 3 216 349 B1**
(45) Date of publication and mention of the grant of the patent: **04.01.2023**
(21) Application number: 17167341.1
(22) Date of filing: 05.12.2008
(51) Int. Cl.: A23C 9/15, A61P 3/02, A23L 33/19, A23L 33/00, A23C 9/20

(54) **PROTEIN-DENSE MICELLAR CASEIN-BASED LIQUID ENTERAL NUTRITIONAL COMPOSITION**
PROTEINREICHE MIZELLENFÖRMIGE FLÜSSIGE ZUSAMMENSETZUNG AUF CASEINBASIS FÜR DIE ENTERALE ERNÄHRUNG
COMPOSITION NUTRITIONNELLE ENTÉRALE LIQUIDE À BASE DE CASÉINE MICELLAIRE DENSE EN PROTÉINES

(30) Priority: 05.12.2007 WO PCT/NL2007/050626; 09.06.2008 EP 08157877; 09.06.2008 US 59865 P; 19.06.2008 WO PCT/EP2008/004938; 14.11.2008 EP 08169152
(43) Date of publication of application: 13.09.2017
(62) Divisional of application: 08857136.9
(73) Proprietor: N.V. Nutricia, 2712 HM Zoetermeer (NL)
(72) Inventor: MINOR, Marcel, 6704 AA Wageningen (NL); SLIWINSKI, Edward Lucian, 8210 Aarhus (DK); HOTRUM, Natalie Elizabeth, 6721 BJ Bennekom (NL); KIERS, Wynette Hermina Agnes, 6671 GB Zetten (NL); VAN STEENIS, Suzanne, 6704 AK Wageningen (NL); WATERINK, Arjan, 3584 CT Utrecht (NL); DE KORT, Esther Jacqueline, 6708 SE Wageningen (NL)
(74) Representative: Nederlandsch Octrooibureau

(56) References cited:
- EP-A1- 1 424 074
- WO-A1-2008/041219
- JP-A- H08 196 236
- US-A- 5 683 984
- J.F. VÉLEZ-RUIZ AND G.V. BARBOSA-CÁNOVAS: "Rheological Properties of Concentrated Milk as a Function of Concentration, Temperature and Storage Time", JOURNAL OF FOOD ENGINIEERING , vol. 35 1998, pages 177-190, XP002516237, Great Britain Retrieved from the Internet: URL:http://www.sciencedirect.com/science?_ ob=ArticleURL&_udi=B6T8J-3T9YYXH-4&_user=9 87766&_rdoc=1&_fmt=&_orig=search&_sort=d&v iew=c&_acct=C000049880&_version=1&_urlVers ion=0&_userid=987766&md5=c5cb0b25684f2509d c54824aaf6e4ac0 [retrieved on 2009-02-20]
- A.O. KARLSSON ET AL: "Relationship Between Physical Properties of Casein Micelles and Rheology of Skim Milk Concentrate", JOURNAL OF DAIRY SCIENCE., vol. 88, no. 11, 1 November 2005 (2005-11-01), pages 3784-3797, XP055289569, US ISSN: 0022-0302, DOI: 10.3168/jds.S0022-0302(05)73064-2
- HEKKEN D L VAN ET AL: "USE OF COLD MICROFILTRATION TO PRODUCE UNIQUE BETA -CASEIN ENRICHED MILK GELS", DAIRY SCIENCE AND TECHNOLOGY (LE LAIT), EDP SCIENCES, PARIS, FR, vol. 80, no. 1, 1 January 2000 (2000-01-01), pages 69-76, XP009079444, ISSN: 0023-7302, DOI: 10.1051/LAIT:2000108
- HERNANDEZ A ET AL: "Isolation of caseins from whey proteins by microfiltration modifying the mineral balance in skim milk", JOURNAL OF DAIRY SCIENCE, AMERICAN DAIRY SCIENCE ASSOCIATION, US, vol. 92, no. 11, 1 November 2009 (2009-11-01), pages 5357-5362, XP026954777, ISSN: 0022-0302 [retrieved on 2009-11-01]
- Nuala M. Rynne ET AL: "Effect of milk pasteurization temperature and in situ whey protein denaturation on the composition, texture and heat-induced functionality of half-fat Cheddar cheese", INTERNATIONAL DAIRY JOURNAL, vol. 14, no. 11, 1 November 2004 (2004-11-01), pages 989-1001, XP055691396, GB ISSN: 0958-6946, DOI: 10.1016/j.idairyj.2004.03.010

## Description

### FIELD OF THE INVENTION

The present invention is in the field of liquid enteral nutritional compositions.

### BACKGROUND OF THE INVENTION

The present invention relates in general to a shelf-stable liquid enteral composition for providing nutrition, either as a supplement, or as a complete nutrition, comprising a high amount of native micellar casein.

Some patients need nutrition, either as a supplement, or as a complete nutrition, in the smallest volume of liquid. Special care must be taken to their protein levels.

These patients can be cachectic patients or persons suffering from end-stage AIDS, cancer or cancer treatment, severe pulmonary diseases like COPD (chronic obstructive pulmonary disease), tuberculosis and other infection diseases or persons that experienced severe surgery or trauma like burns. Furthermore, persons suffering from disorders in the throat or mouth such as oesophageal cancer or stomatitis and persons having problems with swallowing like dysphagic persons, require special liquid, low-volume nutrition. Also, persons just suffering from reduced appetite or loss of taste, will benefit from low-volume, preferably liquid, food.

These patients can also be elderly persons, in particular frail elderly and elderly at risk of becoming frail. In this regard, although an elderly person's energy needs may be reduced, their ability to consume products may also be diminished. For example, they may have difficulty consuming a product due to, e.g., swallowing difficulties, or due the too large amount of product they need to consume to meet the daily intake of nutrients. Hence, compliance is not optimal, and often, the intake is suboptimal, leading to suboptimal nourishment, and in the end, to malnutrition.

The aforementioned groups of patients may be extremely sensitive to food consistency and to the organoleptic properties of the product such as, for instance viscosity, mouth feel, taste, smell and colour. Also, patients such as cachectic patients, typically suffer from extreme weakness which often prevents them from sitting in a vertical position and from drinking food from a carton or even to suck it from a straw. These patients benefit well from liquid low-volume enteral compositions with a high content of nutrients, in particular proteins.

However, increasing calories and/or proteins in a nutritional liquid composition may increase the overall viscosity of the composition. This can make the liquid nutritional composition difficult to consume or administer, and can also diminish the taste of the nutritional composition. Furthermore, technical difficulties exist in producing a stable, in particular a shelf-stable nutritional liquid composition having a high content of proteins.

Therefore, the problem underlying the present invention is to provide a shelf-stable liquid enteral composition for providing nutrition, either as a supplement, or as a complete nutrition, comprising a high content of an intact protein, as major protein source, in the smallest volume of liquid, and which supports nutrition and well-being in the different patient groups mentioned above, in particular to an elderly person or an ill patient.

Major technical difficulties exists in producing such a shelf-stable liquid enteral nutritional composition with a high content of proteins, in particular intact proteins.

For example, increasing the amount of proteins leads to precipitation and sedimentation of proteins and other ingredients, such as lipids and digestible carbohydrates, which imparts nutrient intake.

Concentrating liquids also increases the chance of undesired interactions between ingredients which reduces stability, especially during heating and long-term storage. Shelf-stable is defined as having a stability of more than 6 months on the shelf under normal storage conditions, i.e. at an ambient temperature of between 18 and 25 °C, and at a standard atmospheric pressure.

Furthermore, increasing the protein content in a nutritional liquid composition may increase the overall viscosity of the composition. This can make the liquid nutritional composition difficult to consume or administer, and can also diminish the taste of the nutritional composition. These phenomena often follow non-linear kinetics and the problems quickly increase in magnitude when the concentration of ingredients is increased above 28 weight%. Therefore, many of the commercial shelf-stable liquid products that are currently available have intact protein levels below about 9 g per 100 ml of product.

A known solution to the problem how to increase the protein content to a higher level without imparting viscosity is replacing part of the total protein by peptides or free amino acids. However, this seriously decreases taste appreciation and therefore voluntary intake of the nutritional composition by the patient group.

On the other hand, many concentrates like condensed milks suffer from an incomplete nutrient profile, too high lactose levels, sticky mouth-feel, high viscosity, extreme sweetness and a high osmotic value, which is not appreciated by the consumer and increases rapidly feelings of fullness and satiety after consumption. This makes that the urge to consume more volume deteriorates rapidly once a small amount of the product has been consumed.

### PRIOR ART

WO 02/098242 A1 (Nestle, 12 December 2002) discloses a calorically dense liquid oral supplement (2.25 kcal/ml) based on a (60:40) soy protein isolate/caseinate mixture with a protein level of 9 g/100 ml (16 En%), 12,25 g/100 ml of fat (49 En%), and 19.7 g/100 ml of digestible carbohydrates (35 En%).

US 5,683,984 (Nestec S.A.) and the corresponding EP patent 0 686 396 B1 teach to replace all of the caseinate in a medium energy nutritional formulation (1 kcal/ml) by native micellar casein to obtain a formulation essentially containing native micellar casein with a low viscosity and a thermal stability to withstand sterilization. It discloses a composition containing a maximum of 7 vol% of native micellar casein. However, the latter document does not teach to replace only part of the caseinate by native micellar casein in a high energy high protein nutritional formulation and the problems that would arise in doing so, nor does it teach the poor shelf and heat stability, nor measures to overcome said problems with the formulations according to our invention.

The commercially available product RESOURCE^{®} 2.0 is a high calorie product from Novartis (2 kcal/ml), based on a mixture of calcium and sodium caseinate as protein source, comprising 9 g/100 ml of proteins (18 En%), 8.7 g/100 ml of fat (39 En%), and 21.4 g/100 ml of digestible carbohydrates (43 En%), and is provided in a 237 ml unit dosage.

The commercially available product VHC^{®} 2.25 is a high calorie product from Nestle (2.25 kcal/ml), based on a mixture of calcium- and potassium caseinate and isolated soy protein as protein source, comprising 9 g/100 ml of proteins (16 En%), 12 g/100 ml of fat (48 En%) and 19.7 g/100 ml of digestible carbohydrates (35 En%), and is provided in a 250 ml unit dosage.

The commercially available product FRESUBIN^{®} 2.0 is a high calorie product from Fresenius (2 kcal/ml), based on unspecified milk proteins as protein source, comprising 10 g/100 ml of proteins (20 En%), 7.8 g/100 ml of fat (35 En%), and 22.5 g/100 ml of digestible carbohydrates (45 En%), and is provided in a 200 ml unit dosage.

The commercially available product PRO-CAL SHOT^{®} is a high calorie product from Vitaflo International Ltd (3.34 kcal/ml), based on skimmed milk powder and sodium caseinate as protein source, comprising 6.7 g/100 ml of proteins (8 En%), 28.2 g/100 ml of fat (76 En%), and 13.4 g/100 ml of digestible carbohydrates (16 en%), and is provided in a 250 ml unit dosage.

The commercially available product TwoCal^{®} HN is a high calorie product from Abbott Laboratories (Ross Nutrition) (2 kcal/ml), based on sodium and calcium caseinate as protein source, comprising 8.4 g/100 ml of proteins (16.7 En%), 8.9 g/100 ml of fat (40.1 En%), and 21.6 g/100 ml of digestible carbohydrates (43.2 En%), and is provided in a 237 ml unit dosage.

WO-2008/041219 (Kerry Group Services International Limited, 10 April 2008) discloses a dry milk protein composition comprising at least 12.5 wt% of a slow digesting milk protein, in particular micellar casein. The milk protein composition is first provided in the form of a powder, which is then used into in beverages, desserts, confectionary, baked or dairy products, without pasteurisation or sterilisation. A liquid high protein shake containing 11.4 % Ultra Bio-M^{™} (about 8 weight% of protein) is exemplified. A high protein content of more than 10 weight% and the associated problems were not addressed.

### SUMMARY OF THE INVENTION

The present invention provides a liquid enteral nutritional composition with a high protein content, designed to meet the nutritional needs of persons in need thereof, in particular elderly and patients with certain disease states. In one aspect, the present invention provides a liquid enteral nutritional composition comprising to a large extent native micellar casein, designed to meet the nutritional needs of persons in need thereof, in particular elderly and patients with certain disease states. The composition provides an increased amount of protein per unit volume while providing a sufficiently low viscosity to allow the composition to be easily consumed orally or be administered by tube. In addition, the taste of the composition is not diminished. The invention is as defined in the appended claims.

Micellar casein, also referred to as native micellar casein, refers to casein in the form of micelles. It is a high quality milk protein and naturally occurring in milk in a concentration of about 2.6 g/100 ml (Dairy Science and Technology, Walstra et al., CRC Press, 2006). It is concentrated by a process that does not, or does not substantially denature the casein proteins and it is marketed as Micellar Casein Isolate (MCI). Fresh skim milk is subjected to a microfiltration process, in much the same process used to concentrate whey protein, to produce a pure, substantially undenaturated milk protein with its native structure. The resulting material contains between 90 % and 95 %, preferably more than 95 % by weight of micellar casein, the rest mainly being whey protein and other non-protein nitrogen and other constituents, such as lactose and inorganic salts, in particular calcium phosphate. The casein micelles generally have a hydrodynamic radius of 40 to 400 nm, a molecular weight of 10⁶ to 10⁹ Dalton and a calcium: phosphorous weight ratio of 1.4 to 2.4. It has an intrinsic low viscosity and a liquid composition comprising said MCI is therefore easy to drink.

In contrast, casein, as it is used in the context of this invention refers to the curd form of casein, having lost its native micellar structure. It is bound to a metal, such as sodium, calcium and magnesium.

Within the context of this invention, it is understood that micellar casein may also be provided by other milk protein sources, such as, for instance, sources with essentially preserve the natural 80:20 ratio of casein to whey, such as Milk Protein Concentrate (MPC), which is a powder product usually prepared by ultrafiltration with an average protein content of about 80 weight%, Milk Protein Isolate (MPI), a powder product usually prepared by precipitation with an average protein content of more than 85 weight%, and skimmed concentrated milk.

A problem associated with the use of micellar casein isolate in the production of liquid enteral nutritional compositions with a high protein content is the dissolution of the large amounts of protein powders in the small quantity of water. A viscous intermediate state is produced which is difficult to handle and to process. Especially in highly protein-dense formulations the viscosity may be too high to pump, heat, cool or homogenize the solution, all processes which are necessary to obtain the final product. It is obvious that only the final heat-treated (by sterilisation) product needs to have a low viscosity such that it may be consumed orally or by tube. A further problem associated with the use of micellar casein in the production of liquid enteral nutritional compositions with a high protein content and further containing acids, in particular citric acid, is the formation of calcium-acid complexes, such as calcium citrate. In particular citric acid is added to the composition to adjust the pH and also to adjust Ca-ion activity. A certain Ca-ion activity is beneficial to maintain a desired viscosity of the composition during processing of the composition, e.g. during pasteurisation and/or sterilisation. Calcium, originating from the micellar casein, tends to react with acid, in particular the citric acid, thus forming calcium citrate crystals, which precipitate when the acidity of the composition increases over time (pH lowering), giving rise to a poor shelf stability. Already at a pH of 6.9, the formation of Ca-citrate crystals is progressing. On the other hand, a certain Ca-ion activity is beneficial to maintain a desired viscosity of the composition during processing of the composition, e.g. during pasteurisation and/or sterilisation. In particular a certain Ca-ion activity is beneficial to prevent a viscosity increase during heating. Thus, besides shelf stability, it is a problem to arrive at a proper viscosity when using micellar casein.

These problems have now surprisingly been solved by the inventors using a specific method, in a particular embodiment for a composition comprising micellar casein and caseinate, as defined in the appended claims.

In the context of this invention, the term "at least" also includes the starting point of the open range. For example, an amount of "at least 95 weight%" means any amount equal to 95 weight % or above.

In the context of this invention, enteral means orally or by tube.

In the context of this invention, the % of total energy is also abbreviated as En%; En% is thus short for energy percentage and represents the relative amount that a constituent contributes to the total caloric value of the composition.

In the context of this invention, the term "about" indicates that a certain deviation is allowed from a cited value, the magnitude thereof being determined by *inter alia* the accuracy of the determination method. Typically, such a deviation is 10 %.

In the context of this invention, "non-hydrolysed" proteins is equivalent to "intact" proteins, meaning that the proteins have not, or not substantially, been subjected to an hydrolysis process. However, minor amounts of hydrolysed proteins may be present in the source of non-hydrolysed proteins, or may be added to the formulation, such as additional amino acids, such as for example branched chain amino acids, for example leucine, isoleucine, valine and the like. In this context, "minor" should be understood as an amount of about 10 weight% or less, based on total protein.

In the context of this invention, intact milk protein is defined as a milk protein in its native state originating from milk.

In the context of this invention, it is understood that "liquid" refers to a water-based composition, such as a solution or a suspension, having a viscosity of 200 mPa.s or less, as determined at 20 °C in a rotational rheometer at a shear rate of 100 s⁻¹. A value of about 200 mPa.s is herewith defined as an empirical upper viscosity limit, above which a liquid system has an unacceptably high viscosity to be readily drinkable. It is preferred to provide a composition having a viscosity of less than 200 mPa.s, more preferably 150 mPa.s or less.

In the context of this application, "major" is to be interpreted as at least 70 weight%, more preferably at least 80 weight%, most preferably at least 90 weight%.

The invention will now be further elucidated by describing the preferred embodiments of the present invention in more detail.

### DETAILED DESCRIPTION OF THE INVENTION

### Protein

According to the present invention, a liquid enteral nutritional composition is provided comprising 14 to 18 g of protein per 100 ml of the composition.

The liquid enteral nutritional composition of the invention comprises at most 30 weight% of caseinate, based on the total weight of the protein.

The high levels of protein are beneficial for patients who may not be physically capable of receiving a large volume, for example, fluid restricted patients. Such patients can be given a reduced level of fluid while still receiving a required amount of nutritional support per day. The composition may be used as a complete nutrition, in addition to or as a replacement for a normal meal consumption. The composition may also be used as a supplement, in addition to normal meal consumption, when the uptake of fats and carbohydrates is of less concern.

According to another embodiment of the present invention, the composition has an energy density of at least 0.50 kcal/ml, more preferably at least 1.0 kcal/ml, particularly at least 1.5 kcal/ml of composition.

According to another embodiment of the present invention, the composition has an energy density of less than 2.0 kcal/ml. Although the composition has a high energy density, it also has a sufficiently low viscosity to allow it to be consumed by persons that may have difficulty swallowing products or those that are tube fed.

The amount of native micellar casein in the liquid nutritional composition according to the invention is at least 70 weight% based on the total weight of the protein present in the liquid nutritional composition.

As aforementioned, the composition of the present invention does not contain large amounts of proteins other than micellar casein and at most 30 weight% of caseinate. In a further embodiment of the present invention, the composition may comprise less than or equal to 5 weight% of whey of the total protein present in the liquid nutritional composition.

In one embodiment of the present invention, Na-caseinate, Mg-caseinate, K-caseinate or any mixture thereof or combinations thereof such as Na/K-caseinate and Na/Mg caseinate are used as the source of caseinate. Preferably, Ca-caseinate, or a caseinate comprising Ca is not used, as the micellar casein already contains a sufficient amount of calcium, and the formation of further calcium crystals should be avoided. Preferably, Na-caseinate is used.

The composition according to the invention is designed to either supplement a person's diet or to provide complete nutritional support. Hence, the composition according to the invention may further comprise at least fat and/or carbohydrate and/or a source of vitamins and minerals and/or a source of indigestible carbohydrates. Preferably, the composition according the invention is a nutritionally complete composition.

### Fat

In one embodiment the present liquid enteral nutritional composition further comprises fat. The amount of fat may range between 5 and 95 %, preferably between 10 - 70 %, more preferably between 20 to 40 %, relative to the total energy content of the composition.

With regard to the type of fat, a wide choice is possible, as long as the fat is of food quality.

The fat may either be an animal fat or a vegetable fat or both. Although animal fats such as lard or butter have essentially equal caloric and nutritional values and can be used interchangeably, vegetable oils are highly preferred in the practice of the present invention due to their readily availability, ease of formulation, absence of cholesterol and lower concentration of saturated fatty acids. In one embodiment, the present composition comprises rapeseed oil, corn oil and/or sunflower oil.

The fat may include a source of medium chain fatty acids, such as medium chain triglycerides (MCT, mainly 8 to 10 carbon atoms long), a source of long chain fatty acids, such as long chain triglycerides (LCT) and phospholipid-bound fatty acids such as phospholipid-bound EPA or DHA, or any combination of the two types of sources. MCTs are beneficial because they are easily absorbed and metabolized in a metabolically-stressed patient. Moreover, the use of MCTs will reduce the risk of nutrient malabsorption. LCT sources, such as canola oil, rapeseed oil, sunflower oil, soybean oil, olive oil, coconut oil, palm oil, linseed oil, marine oil or corn oil are beneficial because it is known that LCTs may modulate the immune response in the human body.

In one specific embodiment, the fat comprises 30 to 60 weight% of animal, algal or fungal fat, 40 to 70 weight% of vegetable fat and optionally 0 to 20 weight% of MCTs based on total fat of the composition. The animal fat preferably comprises a low amount of milk fat, i.e. lower than 6 weight%, especially lower than 3 weight% based on total fat. In particular, a mixture of corn oil, egg oil, and/or canola oil and specific amounts of marine oil is used. Egg oils, fish oils and algal oils are a preferred source of non-vegetable fats. Especially for compositions that are to be consumed orally, in order to prevent formation of off-flavours and to decrease a fishy after-taste, it is recommended to select ingredients that are relatively low in docosahexanoic acid (DHA), i.e. less than 6 weight%, preferably less than 4 weight% based on total fat. Marine oils containing DHA are preferably present in the composition according to the invention in an amount lower than 25 weight%, preferably lower than 15 weight% based on total fat. On the other hand, inclusion of eicosapentanoic acid (EPA) is highly desirable for obtaining the maximum health effect. Therefore, in another embodiment, the amount of EPA may range between 4 weight% and 15 weight%, more preferably between 8 weight% and 13 weight% based on total fat. The weight ratio EPA:DHA is advantageously at least 6:4, for example between 2:1 and 10:1. In yet another embodiment, the amount of EPA is very low, such as 0.1 to 1 weight%, preferably 0.3 weight% or 0.6 weight%, based on total fat.

Also, the liquid nutritional composition according to the invention may beneficially comprise an emulsifier. Commonly known emulsifiers may be used and generally the emulsifier contributes to the energy content of the fat in said composition.

### Digestible carbohydrate

In one embodiment of the present invention, the liquid nutritional composition according to the invention further comprises a digestible carbohydrate. Preferably, the digestible carbohydrate provides at least 40 % of the total energy content of the composition according to the invention. The digestible carbohydrate may comprise either simple or complex carbohydrates, or any mixture thereof. Suitable for use in the present invention are glucose, fructose, sucrose, lactose, trehalose, palatinose, corn syrup, malt, maltose, isomaltose, partially hydrolysed corn starch, maltodextrins, glucose oligo- and poly-saccharides.

The composition of the digestible carbohydrate preferably is such that high viscosities, excessive sweetness, excessive browning (Maillard reactions) and excessive osmolarities are avoided. Acceptable viscosities and osmolarities may be achieved by adjusting the average chain length (average degree of polymerisation, DP) of the digestible carbohydrates between 1.5 and 6, preferably between 1.8 and 4. In order to avoid excessive sweetness, the total level of sucrose and fructose is less than 52 % and preferably less than 40 % of the weight of the carbohydrate, especially of the digestible carbohydrate. Long-chain digestible carbohydrates such as starch, starch fractions and mild starch hydrolysates (DP ≥ 6, DE < 20), may also be present, preferably in an amount of less than 25 weight%, especially less than 15 weight% of the digestible carbohydrate, and less than 6 g/100 ml, preferably less than 4 g/100 ml of the total liquid enteral composition according to the invention.

In one embodiment of the present invention, the digestible carbohydrate includes maltodextrose with a high DE (dextrose equivalent). In one embodiment the digestible carbohydrate includes maltodextrose with a DE of > 20, preferably > 30 or even > 40, such as a DE of about 47. Surprisingly, the use of maltodextrose leads to few or no Maillard reaction products upon heating. Without being bound to any explanation, this effect might be attributed to the fact that the compact micellar structure of the micellar casein offers few lysine reaction sites for a Maillard reaction. In one embodiment of the present invention, the digestible carbohydrate includes maltodextrose with a high DE in an amount of at least 35 weight%, preferably at least 50 weight%, preferably at least 65 weight%, preferably at least 90 weight% of the total weight of digestible carbohydrate. In one embodiment of the present invention, the digestible carbohydrate includes maltodextrose with a low DE of 2 to 20. In one embodiment of the present invention, the digestible carbohydrate includes maltodextrose with a low DE of 2 to 10, preferably with a low DE of about 2. In one embodiment of the present invention, the digestible carbohydrate includes maltodextrose with a low DE in an amount of less than 35 weight%, preferably less than 20 weight%, preferably less than 10 weight% of the digestible carbohydrate. Maltodextrose with a low DE may also be referred to as maltodextrine. In another embodiment of the present invention, the digestible carbohydrate includes maltodextrose with a high DE, preferably a DE of > 20, preferably > 30 or even > 40, most preferably a DE of about 47 in combination with maltodextrose with a low DE, preferably a low DE of 2 to 20, more preferably a low DE of 2 to 10, most preferably with a low DE of about 2. As is known, maltodextrose with a low DE, such as of about 2, gives rise to a high viscosity. Maltodextrose with a high DE, such as of about 47 gives rise to a low viscosity, but is very sweet. The combination of both maltodextroses optimizes the balance between sweetness and viscosity. In one embodiment of the present invention, the digestible carbohydrate includes at least 65 weight%, preferably at least 90 weight%, based on total weight of digestible carbohydrate of maltodextrose with a DE >40, preferably with a DE of about 47 and 0 to 10 weight% of maltodextrose with a DE 2 to 10, preferably with a DE of about 2.

In another embodiment of the present invention, the digestible carbohydrate includes trehalose. As was indicated, it is one of the main objects of the invention to provide a nutritional composition with a low viscosity. Sucrose is very well suited for such purpose, but gives rise to very sweet compositions, which are in general disliked by the consumer. Maltodextrose with a low DE, such as of about 2, does not suffer from the latter drawback, but gives rise to a high viscosity. Maltodextrose with a high DE, such as of about 47 gives rise to a low viscosity, but is again very sweet, and gives further rise to the undesired Maillard reactions. Trehalose is a preferred choice of carbohydrate, as it gives rise to a low viscosity, no undesired Maillard reactions and it has a sweetness about half of that of sucrose. In one embodiment of the present invention, the digestible carbohydrate includes trehalose in an amount of 20 % to 60 % of the weight of the carbohydrate, in an amount of 20 % to 45 %, more preferably in an amount of 25 % to 45 % of the weight of the digestible carbohydrate.

### Vitamins and minerals

The composition according to the invention may contain a variety of vitamins and minerals. Overall, the composition according to the invention preferably includes at least 100 % of the United States Recommended Daily Allowance (USRDA) of vitamins and minerals in a one litre portion.

In one embodiment of the present invention, the composition according to the invention provides all necessary vitamins and minerals. For example, the composition according to the invention preferably provides 6 mg of zinc per 100 ml of the composition which is beneficial for tissue repair in a healing patient. Preferably, the composition according to the invention (also) provides 25 mg of vitamin C per 100 ml of the composition to aid patients with more severe healing requirements. Further, preferably, the composition according to the invention (also) provides 2.25 mg iron per 100 ml of the composition. Iron is beneficial in maintaining bodily fluids as well as circulatory system functions in an elderly patient.

In another embodiment of the present invention, the amount of divalent ions ranges between 170 mg/100 ml and 230 mg/100 ml and preferably between 180 mg/100 ml and 220 mg/100 ml. Preferably, the amount of calcium ranges between 155 mg/100 ml and 185 mg/100 ml and preferably between 160 mg/100 ml and 180 mg/100 ml. The phosphorus content can be above 10 mg per g of protein, with a calcium to phosphorus weight ratio between 1.0 and 2.0, preferably between 1.1 and 1.7. Carnitin may advantageously be present in an amount of 8 mg/100 ml to 1000 mg/100 ml, preferably 10 mg/100 ml to 100 mg/100 ml of composition; it may have the form of carnitin, alkyl carnitin, acyl carnation or mixtures thereof. Organic acids are preferably present at a level of between 0.1 g/100 ml to 0.6 g/100 ml, especially 0.25 g/100 ml to 0.5 g/100 ml. These acids include short fatty acids such as acetic acid, hydroxy acids such as lactic acid, gluconic acid, and preferably polyvalent hydroxy acids, such as malic acid and citric acid. In one embodiment of the present invention, the present composition also comprises citric acid.

### Non-digestible carbohydrates

The liquid enteral nutritional composition according to the invention may optionally be fortified with non-digestible carbohydrates (dietary fibres) such as fructo-oligosaccharides or inulin. In an embodiment of the present invention, the composition according to the invention comprises 0.5 g/100 ml to 6 g/100 ml of non-digestible carbohydrates. The dietary fibres include non-digestible oligosaccharides having a DP of 2 to 20, preferably 2 to 10. More preferably, these oligosaccharides do not contain substantial amounts (less than 5 weight%) of saccharides outside these DP ranges, and they are soluble. These oligosaccharides may comprise fructo-oligosaccharides (FOS), trans-galacto-oligosaccharides (TOS), xylo-oligosaccharides (XOS), soy oligosaccharides, and the like. Optionally, also higher molecular weight compounds such as inulin, soy polysaccharides, acacia polysaccharides (acacia fibre or arabic gum), cellulose, resistant starch and the like may be incorporated in the composition according to the invention. The amount of insoluble fibre such as cellulose is preferably lower than 20 weight% of the dietary fibre fraction of the composition according to the invention, and/or below 0.6 g/100 ml. The amount of thickening polysaccharides such as carrageenans, xanthans, pectins, galactomannans and other high molecular weight (DP > 50) indigestible polysaccharides is preferably low, i.e. less than 20 % of the weight of the fibre fraction, or less than 1 g/100 ml. Instead, hydrolysed polysaccharides such as hydrolysed pectins and galactomannans can advantageously be included.

A preferred fibre component is an indigestible oligosaccharide with a chain length (DP) of 2 to 10, for example Fibersol^{®} (resistant oligoglucose), in particular hydrogenated Fibersol^{®}, or a mixture of oligosaccharides having a DP of 2 to 10, such as fructo-oligosaccharides or galacto-oligosaccharides, which may also contain a small amount of higher saccharides (e.g. with a DP of 11 to 20). Such oligosaccharides preferably comprise 50 weight% to 90 weight% of the fibre fraction, or 0.5 g/100 ml to 3 g/100 ml of the composition according to the invention. Other suitable fibre components include saccharides that have only partial digestibility.

In a particular embodiment, the composition according to the invention comprises one or more of fructo-oligosaccharides, inulin, acacia polysaccharides, soy polysaccharaides, cellulose and resistant starch.

In another embodiment of the present invention, the composition according to the invention may comprise a mixture of neutral and acid oligosaccharides as disclosed in WO 2005/039597 (N.V. Nutricia).

More in particular, the acid oligosaccharide has a degree of polymerization (DP) between 1 and 5000, preferably between 1 and 1000, more preferably between 2 and 250, even more preferably between 2 and 50, most preferably between 2 and 10. If a mixture of acid oligosaccharides with different degrees of polymerization is used, the average DP of the acid oligosaccharide mixture is preferably between 2 and 1000, more preferably between 3 and 250, even more preferably between 3 and 50. The acid oligosaccharide may be a homogeneous or heterogeneous carbohydrate. The acid oligosaccharides may be prepared from pectin, pectate, alginate, chondroitine, hyaluronic acids, heparin, heparane, bacterial carbohydrates, sialoglycans, fucoidan, fucooligosaccharides or carrageenan, and are preferably prepared from pectin or alginate. The acid oligosaccharides may be prepared by the methods described in WO 01/603678. The acid oligosaccharide is preferably prepared from high methoxylated pectin, which is characterized by a degree of methoxylation above 50%. As used herein, "degree of methoxylation" (also referred to as DE or "degree of esterification") is intended to mean the extent to which free carboxylic acid groups contained in the polygalacturonic acid chain have been esterified (e.g. by methylation). The acid oligosaccharides are preferably characterized by a degree of methoxylation above 20%, preferably above 50 % even more preferably above 70%. Preferably the acid oligosaccharides have a degree of methylation above 20%, preferably above 50 % even more preferably above 70%. The acid oligosaccharide is preferably administered in an amount of between 10 mg and 100 gram per day, preferably between 100 mg and 50 grams per day, even more between 0.5 and 20 gram per day.

The term neutral oligosaccharides as used in the present invention refers to saccharides which have a degree of polymerization of monose units exceeding 2, more preferably exceeding 3, even more preferably exceeding 4, most preferably exceeding 10, which are not or only partially digested in the intestine by the action of acids or digestive enzymes present in the human upper digestive tract (small intestine and stomach) but which are fermented by the human intestinal flora and preferably lack acidic groups. The neutral oligosaccharide is structurally (chemically) different from the acid oligosaccharide. The term neutral oligosaccharides as used in the present invention preferably refers to saccharides which have a degree of polymerization of the oligosaccharide below 60 monose units, preferably below 40, even more preferably below 20, most preferably below 10. The term monose units refers to units having a closed ring structure, preferably hexose, e.g. the pyranose or furanose forms. The neutral oligosaccharide preferably comprises at least 90%, more preferably at least 95% monose units selected from the group consisting of mannose, arabinose, fructose, fucose, rhamnose, galactose, □-D-galactopyranose, ribose, glucose, xylose and derivatives thereof, calculated on the total number of monose units contained therein. Suitable neutral oligosaccharides are preferably fermented by the gut flora. Preferably the oligosaccharide is selected from the group consisting of: cellobiose (4-O-β-D-glucopyranosyl-D-glucose), cellodextrins ((4-O-β-D-glucopyranosyl)ₙ-D-glucose), B-cyclodextrins (Cyclic molecules of α-1-4-linked D-glucose; α-cyclodextrin-hexamer, β-cyciodextrin-heptamer and γ-cyclodextrin-octamer), indigestible dextrin , gentiooligosaccharides (mixture of β-1-6 linked glucose residues, some 1-4 linkages), glucooligosaccharides (mixture of α-D-glucose), isomaltooligosaccharides (linear α-1-6 linked glucose residues with some 1-4 linkages), isomaltose (6-O-α-D-glucopyranosyl-D-glucose); isomaltriose (6-O-α-D-glucopyranosyl-(1-6)-α-D-glucopyranosyl-D-glucose), panose (6-O-α-D-glucopyranosyl-(1-6)-α-D-glucopyranosyl-(1-4)-D-glucose), leucrose (5-O-α-D-glucopyranosyl-D-fructopyranoside), palatinose or isomaltulose (6-O-α-D-glucopyranosyl-D-fructose), theanderose (O-α-D-glucopyranosyl-(1-6)-O-a-D-glucopyranosyl-(1-2)-B-D-fructofuranoside), D-agatose, D-*lyxo*-hexulose, lactosucrose (O-β-D-galactopyranosyl-(1-4)-O-α-D-glucopyranosyl-(1-2)-β-D-fructofuranoside), α-galactooligosaccharides including raffinose, stachyose and other soy oligosaccharides (O-α-D-galactopyranosyl-(1-6)-α-D-glucopyranosyl-β-D-fructofuranoside), β-galactooligosaccharides or transgalacto-oligosaccharides (β-D-galactopyranosyl-(1-6)-[β-D-glucopyranosyl]ₙ-(1-4) α-D glucose), lactulose (4-O-β-D-galactopyranosyl-D-fructose), 4'-galatosyllactose (O-D-galactopyranosyl-(1-4)-O-β-D-glucopyranosyl-(1-4)-D-glucopyranose), synthetic galactooligosaccharide (neogalactobiose, isogalactobiose, galsucrose, isolactosel, II and III), fructans - Levan-type (β-D-(2→6)-fructofuranosyl)ₙ α-D-glucopyranoside), fructans - Inulin-type (β-D-((2→1)-fructofuranosyl)ₙ α-D-glucopyranoside), 1 f-β-fructofuranosylnystose (β-D-((2→1)-fructofuranosyl)ₙ B-D-fructofuranoside), xylooligosaccharides (B-D-((1→4)-xylose)ₙ, lafinose, lactosucrose and arabinooligosaccharides.

According to a further preferred embodiment the neutral oligosaccharide is selected from the group consisting of fructans, fructooligosaccharides, indigestible dextrins galactooligosaccharides (including transgalactooligosaccharides), xylooligosaccharides, arabinooligosaccharides, glucooligosaccharides, mannooligosaccharides, fucooligosaccharides and mixtures thereof. Most preferably, the neutral oligosaccharide is selected from the group consisting of fructooligosacchararides, galactooligosaccharides and transgalactooligosaccharides. Suitable oligosaccharides and their production methods are further described in Laere K.J.M. (Laere, K.J.M., Degradation of structurally different non-digestible oligosaccharides by intestinal bacteria: glycosylhydrolases of Bi. adolescentis. PhD-thesis (2000), Wageningen Agricultural University, Wageningen, The Netherlands).

Transgalactooligosaccharides (TOS) are for example sold under the trademark Vivinal^{™} (Borculo Domo Ingredients, Netherlands). Indigestible dextrin, which may be produced by pyrolysis of corn starch, comprises α(1→4) and α(1→6) glucosidic bonds, as are present in the native starch, and contains 1→2 and 1→3 linkages and levoglucosan. Due to these structural characteristics, indigestible dextrin contains well-developed, branched particles that are partially hydrolysed by human digestive enzymes. Numerous other commercial sources of indigestible oligosaccharides are readily available and known to skilled person. For example, transgalactooligosaccharide is available from Yakult Honsha Co., Tokyo, Japan. Soybean oligosaccharide is available from Calpis Corporation distributed by Ajinomoto U.S.A. Inc., Teaneck, N.J.

In a further preferred embodiment, the composition according to the invention comprises an acid oligosaccharide with a DP between 2 and 250, prepared from pectin, alginate, and mixtures thereof ; and a neutral oligosaccharide, selected from the group of fructans, fructooligosaccharides, indigestible dextrins, galactooligosaccharides including transgalactooligosaccharides, xylooligosaccharides, arabinooligosaccharides, glucooligosaccharides, mannooligosaccharides, fucooligosaccharides, and mixtures thereof.

In a further preferred embodiment the composition according to the invention comprises two chemically distinct neutral oligosaccharides. It was found that the administration of acid oligosaccharides combined with two chemically distinct neutral oligosaccharides provides an optimal synergistic immune stimulatory effect.

Preferably the composition according to the invention comprises:
- an acid oligosaccharides as defined above ;
- a galactose-based neutral oligosaccharide (of which more than 50 % of the monose units are galactose units), preferably selected from the group consisting of galactooligosaccharide and transgalactooligosaccharide; and
- a fructose and/or glucose based neutral oligosaccharide (of which more than 50% of the monose units are fructose and/or glucose, preferably fructose units), preferably inulin, fructan and/or fructooligosaccharide, most preferably long chain fructooligosaccharide (with an average DP of 10 to 60).

The mixture of acid- and neutral oligosaccharides is preferably administered in an amount of between 10 mg and 100 gram per day, preferably between 100 mg and 25 grams per day, even more preferably between 0.5 and 20 gram per day.

### Viscosity and osmolarity

In the context of this invention, the viscosity is measured in a rotational rheometer using a cone-plate geometry at 20 °C at a shear rate of 100 s⁻¹.

In one embodiment of the present invention, the viscosity of the liquid enteral nutritional composition is less than about 200 mPa.s, preferably less than 150 mPa.s, preferably less than 100 mPa.s. A low viscosity is ideal for orally administering the liquid enteral nutritional composition according to the invention because a person may easily consume a serving having a low viscosity such as that displayed by the present invention. This is also ideal for unit dosages that are tube fed.

In one embodiment of the present invention, the osmolarity of the composition is preferably lower than 900 mOsm/l, more preferably lower than 800 mOsm/l, most preferable lower than 700 mOsm/l.

In one embodiment of the present invention, the density of the composition ranges between 1.05 g/ml and 1.20 g/ml, especially between 1.10 g/ml and 1.18 g/ml.

### Dosage unit

The liquid enteral nutritional composition according to the invention may have the form of a complete food, i.e. it can meet all nutritional needs of the user. As such, it preferably contains 1200 to 2500 kcal per daily dosage. The daily dosage amounts are given with respect to a daily energy supply of 2000 kcal to a healthy adult having a body weight of 70 kg. For persons of different condition and different body weight, the levels should be adapted accordingly. It is understood that the average daily energy intake preferably is about 2000 kcal. The complete food can be in the form of multiple dosage units, e.g. from 4 (250 ml/unit) to 20 (50 ml/unit) per day for an energy supply of 2000 kcal/day using a liquid enteral nutritional composition according to the invention of 2.0 kcal/ml.

The liquid enteral nutritional composition can also be a food supplement, for example to be used in addition to a non-medical food. Preferably as a supplement, the liquid enteral nutritional composition contains per daily dosage less than 1500 kcal, in particular as a supplement, the liquid enteral nutritional composition contains 400 to 1000 kcal per daily dose. The food supplement can be in the form of multiple dosage units, e.g. from 2 (250 ml/unit) to 10 (50 ml/unit) per day for an energy supply of 1000 kcal/day using a liquid enteral nutritional composition according to the invention of 2.0 kcal/ml.

In one embodiment of the present invention, a unit dosage comprises any amount of the liquid enteral nutritional composition according to the invention between 10 ml and 250 ml, the end values of this range included, preferably any amount between 25 ml and 200 ml, the end values of this range included, more preferably any amount between 50 ml and 150 ml, the end values of this range included, most preferably about 125 ml. For example, a person receiving 50 ml unit dosages can be given 10 unit dosages per day to provide nutritional support using a liquid enteral nutritional composition according to the invention of 2.0 kcal/ml. Alternatively a person receiving 125 ml unit dosages can be given 4 or 5 or 6 or 7 or 8 unit dosages per day to provide nutritional support using a liquid enteral nutritional composition according to the invention of 2.0 kcal/ml. Such small dosage units are preferred because of better compliance.

In one embodiment of the present invention, the composition is provided in a ready to use liquid form and does not require reconstitution or mixing prior to use. The composition according to the invention can be tube fed or administered orally. For example, the composition according to the invention can be provided in a can, on spike, and hang bag. However, a composition may be provided to a person in need thereof in powder form, suitable for reconstitution using an aqueous solution or water such that the composition according to the invention is produced. Thus in one embodiment of the present invention, the present composition is in the form of a powder, accompanied with instructions to dissolve or reconstitute in an aqueous composition or water to arrive at the liquid nutritional enteral composition according to the present invention. In one embodiment of the present invention, the present liquid nutritional enteral composition may thus be obtained by dissolving or reconstituting a powder, preferably in an aqueous composition, in particular water.

In one embodiment of the present invention, the composition according to the invention is packaged. The packaging may have any suitable form, for example a block-shaped carton, e.g. to be emptied with a straw ; a carton or plastic beaker with removable cover; a small-sized bottle for example for the 80 ml to 200 ml range, and small cups for example for the 10 ml to 30 ml range. Another suitable packaging mode is inclusion of small volumes of liquid (e.g. 10 ml to 20 ml) in edible solid or semi-solid hulls or capsules, for example gelatine-like coverings and the like. Another suitable packaging mode is a powder in a container, e.g. a sachet, preferably with instructions to dissolve or reconstitute in an aqueous composition or water.

### Preparation

The liquid enteral nutritional composition according to the invention may be prepared by an inventive process comprising a step wherein an aqueous protein solution in which all or a major part of said protein comprises native micellar casein, is subjected to an evaporation step.

Firstly, a liquid protein composition is prepared. This may be done by sequentially or simultaneously dissolving micellar casein in powder form (such as MCI) and optionally caseinate in powder form in water. In order to obtain a low-viscosity protein solution which can be processed further, the protein ingredients are dissolved in an excess of water, typically 150 weight% or volume% of what is required for the final composition. Without this excess of water, the protein solution is too thick for homogenization and pasteurisation.

Furthermore, if the liquid enteral nutritional composition is to contain further components, such as carbohydrates, fat and vitamins, a nutritional product may be prepared by subsequently adding the carbohydrates to the protein composition, followed by optionally adding the water-soluble vitamins and other components in one or two stages, mixing, adding the fat, including fat-soluble vitamins, homogenizing, and subjecting the resulting solution to a pasteurization step. The pH may be adjusted. The intermediate enteral nutritional composition is now subsequently concentrated to the desired dry matter concentration by an evaporation step to get rid of the excess of water. Evaporation may be performed at atmospheric pressure at a temperature of typically above 60 °C, or preferably under vacuum at a temperature of typically 60 °C. A higher viscosity may be obtained by this evaporation step. Surprisingly, the viscosity of the resulting solution is still low enough to have a sufficient heat transfer in the product to optimally subject the product to the final heat treatment necessary for providing the product with a long microbial shelf life (sterilization). In this respect, it is noted that the acidity of the composition is very important during the heat-treatment. The pH should be between about 6.6 and 7.2 for the pasteurisation and sterilisation. Typical pasteurisation times are 30 sec at 85 °C. Typical sterilisation times are 4 minutes at 124 °C. Surprisingly, the final heat treatment (sterilisation) decreases the viscosity of the treated composition such that a microbial stable product is obtained with a much lower viscosity than before the sterilisation. Without being bound to any explanation, it is believed that during the extensive final heat treatment (sterilisation), restructuring of the micellar casein into a more compact structure results in a lowering of the viscosity. Surprisingly, the product viscosity did not change substantially during storage over longer periods of time, which provides the product with a long shelf life. Surprisingly, liquid sterilized products, with high protein concentrations could be prepared.

Disclosed herein, but not part of the invention, is a method for preparing the compositions of the invention, comprising a step wherein an aqueous protein solution, obtained by dissolving a micellar casein in powder form, and optionally micellar casein in powder form, is subjected to an evaporation step.

### Effectivity

Disclosed herein, but not part of the invention, is a method of providing nutrition to a person in need thereof, comprising the steps of administering to said person the nutritional composition according to the present invention. Said person may be an elderly person, a person that is in a disease state, a person that is recovering from a disease state, or a person that is malnourished.

In this respect, it is submitted that in the context of this application, an elderly person is a person of the age of 50 or more, in particular of the age of 55 or more, more in particular of the age of 60 or more, more in particular of the age of 65 or more. This rather broad definition takes into account the fact that the average age varies between different populations, on different continents, etc. Most developed world countries have accepted the chronological age of 65 years as a definition of 'elderly' or older person (associated with the age at which one may begin to receive pension benefits), but like many westernized concepts, this does not adapt well to e.g. the situation in Africa. At the moment, there is no United Nations (UN) standard numerical criterion, but the UN agreed cut-off is 60+ years to refer to the older population in Western world. The more traditional African definitions of an elder or 'elderly' person correlate with the chronological ages of 50 to 65 years, depending on the setting, the region and the country.

Disclosed herein, but not part of the invention is the simultaneous or sequential use of micellar casein and optionally caseinate in the manufacture of a liquid nutritional composition according to the present invention for providing enteral nutrition to a person in need thereof.

### EXAMPLES

The following compositions have been prepared (Table 1).

### Example 1

MCI and sodium caseinate were dry mixed and dissolved in demineralised water at ambient temperature to a concentration of 10% w/w protein. The pH was adjusted to 6.8 with citric acid. The solution was homogenized and subsequently concentrated by the help of an evaporator to 18 % protein and a viscosity of 300 mPa.s at 20°C and a shear rate of 100/s. The product was sterilized in glass bottles in the retort (15 minutes at 121 °C). The final product has a small particle size and a low viscosity of 60 mPa.s at 20°C and a shear rate of 100/s.

Without the evaporation step, a paste-like texture was obtained after dissolution of the proteins which could not be processed any further.

Vitamins and minerals amounted to 16 % of RDI.

The compositions 2 to 7 (Table 1) and 8 to 11 (Table 2) were made in a similar way. All compositions are shelf-stable, have desired organoleptic properties, have a very high nutrient density and are effective for a person in need thereof. The compositions 8 to 10 in Table 2 are suitable for use by morbidly obese patients pre- and post-bariatric surgery, as was disclosed in our co-pending application PCT/EP 2008/004938.

### Notes to the tables.

* Micellar Casein Isolate (MCI) contains about 89 weight% of micellar casein and whey with a micellar casein:whey ratio of about 95:5, relative to total dry matter.
* Na-caseinate contains about 96 weight% of casein protein, relative to total dry matter. * At least examples 6-10 are for comparative purposes only.

**Table 1**

| **Component (Amounts per 100 ml of product)** | | | **Example 1** | **Example 2** | **Example 3** | **Example 4** | **Example 5** | **Example 6** | **Example 7** |
|---|---|---|---|---|---|---|---|---|---|
| Energy | | | 72 kcal | 128 kcal | 128 kcal | 160 kcal | 160 kcal | 160 kcal | 160 kcal |
| Protein | | | 100 En% | 53 En% | 53 En% | 40 En% | 40 En% | 30 En% | 30 En% |
| | Protein | | 18 g | 16 g | 16 g | 16 g | 16 g | 12 g | 12 g |
| | MCI* | | 15.75 g | 12.8 g | 15.4 g | 12.8 g | 15.4 g | 10.4 g | 10.4 g |
| | Na-caseinate* | | 2.25 g | 3.2 g | 0.6 g | 3.2 g | 0.6 g | 1.6 g | 1.6 g |
| | *MCI:Na-caseinate* | | *87.5:12.5* | *80:20* | *96:4* | *80:20* | *96:4* | *87:13* | *87:13* |
| | whey | | 0 | 0.6 g | 0.8 g | 0.8 g | 0.8 g | 0.5 g | 0.5 g |
| Fat | | | 0 | 17 En% | 17 En% | 30 En% | 30 En% | 32 En% | 32 En% |
| | Fat (mainly comprising canola oil) | | | 2.4 g | 2.4 g | 5.3 g | 5.3 g | 5.7 g | 5.7 g |
| Carbohydrates | | | 0 | 27 En% | 27 En% | 30 En% | 30 En% | 38 En% | 38 En% |
| | Carbohydrates | | | 8.6 g | 8.6 g | 12 g | 12 g | 15.4 g | 15.4 g |
| | | lactose | | 0.2 g | 0.2 g | 0.2 g | 0.2 g | 0.1 g | 0.1 g |
| | | sucrose | | 3.5 g | 3.5 g | 3.5 g | 3.5 g | 11.8 g | 11.8 g |
| | | maltodextrin (DE 19) | | 4.9 g | 4.9 g | 8.3 g | 8.3 g | 3.5 g | 3.5 g |
| Dietary fibre | | | 0 | 4.0 g | 4.0 g | n.d. | n.d. | n.d. | 3.2 g |
| Viscosity (mPa.s at 20 °C at 100 s⁻¹) | | | 60 | 70 | 70 | 75 | 75 | n.d. | n.d. |

| | | | | | | | | | |
|---|---|---|---|---|---|---|---|---|---|
| n.d. = not determined | | | | | | | | | |

**Table 2**

| **Component (Amounts per 100 ml of product)** | | | **Example 8** | **Example 9** | **Example 10** | **Example 11** |
|---|---|---|---|---|---|---|
| Energy | | | 120 kcal | 108 kcal | 120 kcal | 240 kcal |
| Protein | | | 53 En% | 53 En% | 53 En% | 25 En% |
| | Protein | | 16 g | 13.3 g | 16 g | 15 g |
| | MCl* | | > 15.2 g | > 12.6 g | > 15.2 g | 14 g |
| | Na-caseinate* | | 0 | 0 | 0 | 1 g |
| | *MCI:Na-caseinate* | | *100:0* | *100:0* | *100:0* | 93:7 |
| | whey | | < 0.8 g | < 0.4 g | < 0.8 g | < 0.8 g |
| Fat | | | 18 En% | 18 En% | 18 En% | 35 En% |
| | Fat | | 2.4 g | 2.1 g | 2.4 g | 9.3 g |
| Carbohydrates | | | 29 En% | 29 En% | 29 En% | 40 En% |
| | Carbohydrates | | 8.6 g | 7.8 g | 8.6 g | 24 g |
| | | lactose | 1.4 g | 1.3 g | 1.4 g | 0 g |
| | | sucrose | 4.9 g | 4.4 g | 2.8 g | 12 g |
| | | maltodextrin (DE 19) | 2.3 g | 2.1 g | 2.3 g | 12 g |
| Dietary fibre | | | 3.6 g | 3.2 g | 3.6 g | 0 |
| Viscosity (mPa.s at 20 °C at 100 s⁻¹) | | | n.d. | n.d. | n.d. | 70 |
| Dichtheid | | | n.d. | n.d. | n.d. | 1.16 g/ml |

| | | | | | | |
|---|---|---|---|---|---|---|
| n.d. : not determined | | | | | | |

## Claims

1. A packaged, heat-sterilized liquid enteral nutritional composition comprising 14 to 18 g of protein per 100 ml of the composition, wherein the protein comprises at least 70 weight% of native micellar casein and at most 30 weight% caseinate, based on the total weight of the protein, wherein the combined amount of micellar casein and caseinate is at least 95 weight% based on the total weight of the protein.

2. The liquid enteral nutritional composition according to claim 1, wherein the caseinate is Na-caseinate.

3. The liquid enteral nutritional composition according to any one of the preceding claims, further comprising fat and digestible carbohydrate, said fat providing between 10 to 70 % of the total energy content of the composition, and said carbohydrate providing between 30 to 60 % of the total energy content of the composition.

4. The liquid enteral nutritional composition according to any one of the preceding claims, having an energy density of less than 2.0 kcal/ml.

## Patentansprüche

1. Verpackte, hitzesterilisierte flüssige enterale Nahrungszusammensetzung, umfassend 14 bis 18 g Protein pro 100 ml der Zusammensetzung, wobei das Protein mindestens 70 Gewichts-% eines nativen mizellaren Kaseins und höchstens 30 Gewichts-% Kaseinat basierend auf dem Gesamtgewicht des Proteins umfasst, wobei die kombinierte Menge des mizellaren Kaseins und des Kaseinats mindestens 95 Gewichts-% basierend auf dem Gesamtgewicht des Proteins ist.

2. Flüssige enterale Nahrungszusammensetzung nach Anspruch 1, wobei das Kaseinat Na-Kaseinat ist.

3. Flüssige enterale Nahrungszusammensetzung nach einem der vorhergehenden Ansprüche, die ferner Fett und verdauliche Kohlenhydrate umfasst, wobei das Fett zwischen 10 bis 70% des Gesamtenergiegehalts der Zusammensetzung bereitstellt, und das Kohlenhydrat zwischen 30 bis 60% des Gesamtenergiegehalts der Zusammensetzung bereitstellt.

4. Flüssige enterale Nahrungszusammensetzung nach einem der vorhergehenden Ansprüche, mit einer Energiedichte von weniger als 2,0 kcal/ml.

## Revendications

1. - Composition nutritionnelle entérale liquide, stérilisée par la chaleur, conditionnée, comprenant 14 à 18 g de protéines pour 100 ml de la composition, dans laquelle les protéines comprennent au moins 70 % en poids de caséine micellaire native et au plus 30 % en poids de caséinate, sur la base du poids total des protéines, dans laquelle la quantité combinée de caséine micellaire et de caséinate est d'au moins 95 % en poids sur la base du poids total des protéines.

2. - Composition nutritionnelle entérale liquide selon la revendication 1, dans laquelle le caséinate est le caséinate de Na.

3. - Composition nutritionnelle entérale liquide selon l'une quelconque des revendications précédentes, comprenant en outre des matières grasses et des glucides digestibles, lesdites matières grasses fournissant de 10 à 70 % du contenu énergétique total de la composition, et lesdits glucides fournissant de 30 à 60 % du contenu énergétique total de la composition.

4. - Composition nutritionnelle entérale liquide selon l'une quelconque des revendications précédentes, ayant une densité énergétique de moins de 2,0 kcal/ml.
